# EUROPÄISCHE PATENTSCHRIFT

(11) **EP 2 331 225 B1**
(45) Veröffentlichungstag und Bekanntmachung des Hinweises auf die Patenterteilung: **26.02.2014**
(21) Anmeldenummer: 09783090.5
(22) Anmeldetag: 16.09.2009
(51) Int. Cl.: B01D 3/14, B01D 3/32, B01D 3/42, C07C 213/10, C07C 213/04

(54) **VORRICHTUNGEN UND VERFAHREN ZUR KONTINUIERLICHEN DESTILLATIVEN AUFTRENNUNG EINES GEMISCHES ENTHALTEND EIN ODER MEHRERE ALKANOLAMIN/E**
DEVICES AND METHOD FOR CONTINUOUS DISTILLATIVE SEPARATION OF A MIXTURE CONTAINING ONE OR MORE ALKANOLAMINE(S)
DISPOSITIFS ET PROCÉDÉS DE SÉPARATION PAR DISTILLATION EN CONTINU D'UN MÉLANGE CONTENANT UNE OU PLUSIEURS ALCANOLAMINES

(30) Priorität: 17.09.2008 EP 08164480
(43) Veröffentlichungstag der Anmeldung: 15.06.2011
(73) Patentinhaber: BASF SE, 67056 Ludwigshafen (DE)
(72) Erfinder: SCHMIDT, Willi, 67069 Ludwigshafen (DE); KAIBEL, Gerd, 68623 Lampertheim (DE); GEIßLER, Elke, 67069 Ludwigshafen (DE); REIF, Wolfgang, 67227 Frankenthal (DE); JULIUS, Manfred, 67117 Limburgerhof (DE); PAPE, Frank-Friedrich, 67259 Kleinniedesheim (DE)
(86) Internationale Anmeldenummer: PCT/EP2009/062016
(87) Internationale Veröffentlichungsnummer: WO 2010/031790

(56) Entgegenhaltungen:
- EP-A- 1 443 036
- WO-A-2005/035481
- WO-A-2005/037769

## Beschreibung

Die vorliegende Erfindung betrifft eine Vorrichtung und ein Verfahren zur kontinuierlichen destillativen Auftrennung eines Gemisches enthaltend Monoisopropanolamin (MIPOA), Diisopropanolamin (DIPOA) und Triisopropanolamin (TIPOA).

Für die destillative, z. B. kontinuierliche Zerlegung von Mehrstoffgemischen sind verschiedene Verfahrensvarianten gebräuchlich. Im einfachsten Fall wird das aufzutrennende Gemisch (Zulaufgemisch) in zwei Fraktionen, eine leichtsiedende Kopffraktion und eine schwersiedende Sumpffraktion, zerlegt.

Bei der Auftrennung von Zulaufgemischen in mehr als zwei Fraktionen müssen nach dieser Verfahrensvariante mehrere Destillationskolonnen eingesetzt werden. Um den apparativen Aufwand zu begrenzen, setzt man bei der Auftrennung von Vielstoffgemischen nach Möglichkeit Kolonnen mit flüssigen oder dampfförmigen Seitenabzügen ein.

Die Anwendungsmöglichkeit von Destillationskolonnen mit Seitenabzügen ist jedoch dadurch stark eingeschränkt, dass die an den Seitenabzugsstellen entnommenen Produkte selten oder nie völlig rein sind. Bei Seitenentnahmen im Verstärkungsteil der Kolonne, die üblicherweise in flüssiger Form erfolgen, enthält das Seitenprodukt noch Anteile an leichtsiedenden Komponenten, die über Kopf abgetrennt werden sollen. Entsprechendes gilt für Seitenentnahmen im Abtriebsteil der Kolonne, die meist dampfförmig erfolgen, bei denen das Seitenprodukt noch Hochsiederanteile aufweist.

Die Verwendung von konventionellen Seitenabzugskolonnen ist daher auf Fälle begrenzt, in denen verunreinigte Seitenprodukte zulässig sind.

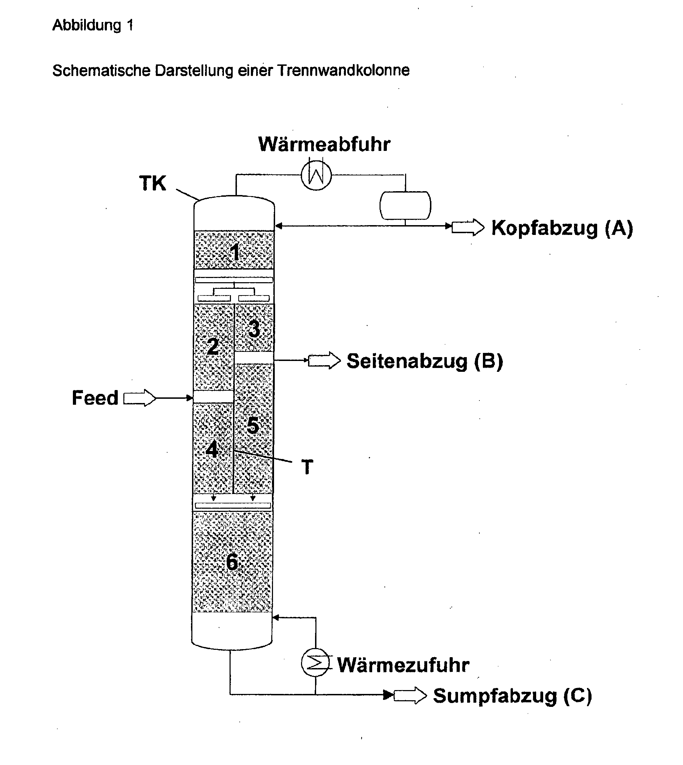

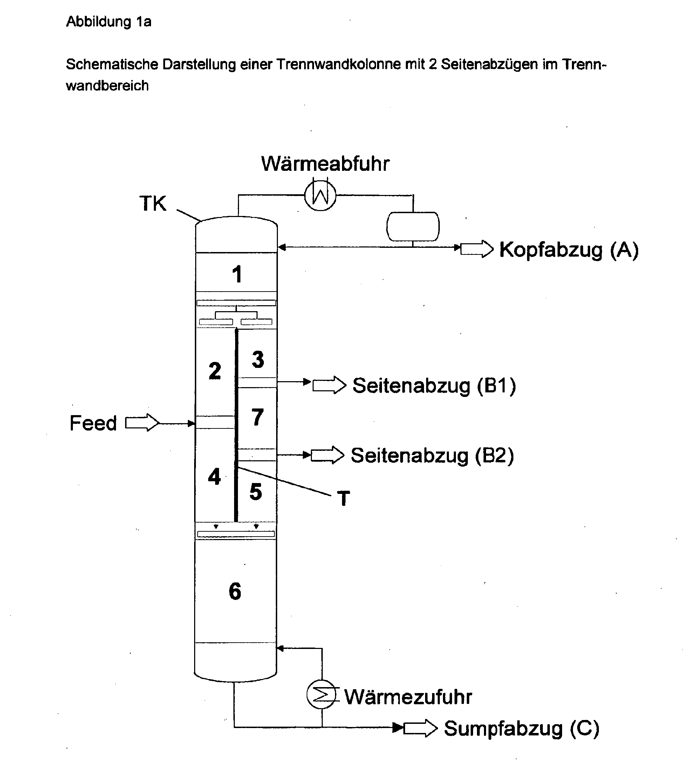

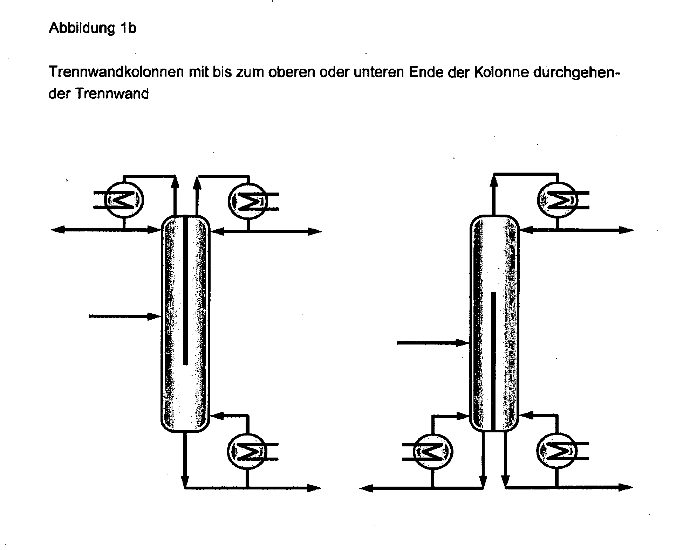

Eine Abhilfemöglichkeit bieten Trennwandkolonnen, bei denen auch die Seitenprodukte in hoher Reinheit gewonnen werden können (siehe z. B. Abbildung 1). Dieser Kolonnentyp ist beispielsweise beschrieben in:
US 2,471,134, US 4,230, 533, EP 122 367 A, EP 126 288 A, EP 133 510 A,
Chem. Eng. Technol. 10, (1987), Seiten 92 - 98,
Chem.-Ing.-Tech. 61, (1989), Nr. 1, Seiten 16 - 25,
Gas Separation and Purification 4 (1990), Seiten 109 - 114,
Process Engineering 2 (1993), Seiten 33 - 34,
Trans IChemE 72 (1994), Part A, Seiten 639 - 644, und
Chemical Engineering 7 (1997), 72 - 76.

Bei dieser Bauart ist im mittleren Bereich oberhalb und unterhalb der Zulaufstelle und der Seitenentnahme eine Trennwand angebracht, die den Zulaufteil 2, 4 gegenüber dem Entnahmeteil 3, 5 abdichtet und in diesem Kolonnenteil eine Quervermischung von Flüssigkeits- und Brüdenströmen unterbindet. Hierdurch verringert sich bei der Auftrennung von Vielstoffgemischen die Zahl der insgesamt benötigten Destillationskolonnen. Wie bei konventionellen Seitenabzugskolonnen können auch bei Trennwandkolonnen Zwischenverdampfer und Zwischenkondensatoren eingesetzt werden. Zwischenkondensatoren werden bevorzugt am oberen Ende der Trennwand oder im gemeinsamen Kolonnenbereich 1 oberhalb der Trennwand angebracht. Zwischenverdampfer werden bevorzugt am unteren Ende der Trennwand oder im gemeinsamen Kolonnenbereich 6 unterhalb der Trennwand vorgesehen.

Eine Trennwandkolonne kann bei gleichem Energieverbrauch auch durch die Anordnung von thermisch gekoppelten Destillationskolonnen ersetzt werden. Eine Beschreibung von thermisch gekoppelten Destillationskolonnen, die in verschiedener apparativer Gestaltung ausgeführt sein können, findet sich ebenfalls in den oben genannten Stellen in der Fachliteratur. Es ist auch möglich, die einzelnen Teilkolonnen komplett mit Verdampfern und Kondensatoren auszurüsten. Dies entspricht einer Trennwandkolonne mit einem Zwischenverdampfer und einem Zwischenkondensator. Ein besonderer Vorteil dieser speziellen Ausgestaltung ist, dass die einzelnen Kolonnen auch bei unterschiedlichen Drücken betrieben werden können. Dies ermöglicht es, zu hohe Temperaturspreizungen zu vermeiden und die Betriebstemperaturen besser an vorgegebene Heiz- und Kühlmedien anzupassen. Die Möglichkeiten für Energieverbundmaßnahmen werden verbessert.

In einer speziellen Ausgestaltung können bei Trennwandkolonnen und thermisch gekoppelten Destillationskolonnen statt einer auch zwei reine Seitenfraktionen entnommen werden. Der Entnahmeteil 3, 5 wird durch einen zwischengeschalteten Kolonnenbereich 7 erweitert (Abbildung 1a). Es ist auch möglich, in den Kolonnenbereichen 1, 3, 7, 5 und 6 oder zwischen den Kolonnenbereichen 1 und 3 sowie 5 und 6 weitere Seitenentnahmen vorzusehen, die jedoch keine völlig reine Fraktionen liefern können.

Eine weitere Bauform von erfindungsgemäß einsetzbaren Trennwandkolonnen sieht vor, die Trennwand durchgehend entweder bis zum oberen oder unteren Ende der Destillationskolonne auszuführen (Abbildung 1 b). Diese Bauform entspricht der Anordnung einer Hauptkolonne mit angeschlossener Seitenkolonne. Bei dieser Ausführungsform sind gegenüber konventionellen Kolonnenanordnungen keine Energie-, dafür aber Investitionskostenvorteile zu erwarten.

Trennwandkolonnen und thermisch gekoppelte Destillationskolonnen bieten gegenüber der Anordnung von konventionellen Destillationskolonnen sowohl hinsichtlich des Energiebedarfs als auch der Investitionskosten Vorteile.

Für die Regelung von Trennwandkolonnen und thermisch gekoppelten Kolonnen werden verschiedene Regelungsstrategien beschrieben. Beschreibungen finden sich in:
US 4,230,533, DE 35 22 234 C2, EP 780 147 A,
Process Engineering 2 (1993), 33 - 34, und
Ind. Eng. Chem. Res. 34 (1995), 2094-2103.

WO 05/035481 A2 (BASF AG) betrifft ein Verfahren zur kontinuierlichen, destillativen Abtrennung von Triethanolamin aus einem durch die Umsetzung von Ammoniak mit Ethylenoxid in flüssiger Phase unter Druck und bei erhöhter Temperatur erhaltenen Stoffgemisch aus Mono-, Di- und Triethanolamin sowie Ethanolamin-Ether und Wasser, indem das Stoffgemisch in zwei Stufen destilliert wird, wobei in der ersten Stufe die Leichtsiederfraktion und die Schwersiederfraktion entnommen und ausgeschleust werden und in der zweiten Stufe die Mittelsiederfraktion mit einem Gehalt an Triethanolamin von > 99,4 Gew.-% und Diethanolamin von < 0,2 Gew.-% destilliert wird.

WO 05/037769 A1 (BASF AG) beschreibt ein Verfahren zur destillativen Auftrennung von Gemischen enthaltend Ethylenamine, indem die Auftrennung in einer oder mehreren Trennwandkolonnen durchgeführt wird und wobei es sich bei den Ethylenaminen insbesondere um Ethylendiamin (EDA), Piperazin (PIP), Diethylentriamin (DETA), Aminoethylethanolamin (AEA) und/oder Monoethanolamin (MEOA) handelt.

Der vorliegenden Erfindung lag die Aufgabe zugrunde, ein verbessertes wirtschaftliches Verfahren zur Auftrennung eines Gemisches enthaltend Monoisopropanolamin (MIPOA), Diisopropanolamin (DIPOA) und Triisopropanolamin (TIPOA) aufzufinden. Die einzelnen Alkanolamine, Monoisopropanolamin (MIPOA), Diisopropanolamin (DIPOA) und Triisopropanolamin (TIPOA), sollten dabei jeweils in hoher Reinheit und unter Einhaltung weiterer Spezifikationsmerkmale, insbesondere Farbqualität, anfallen.

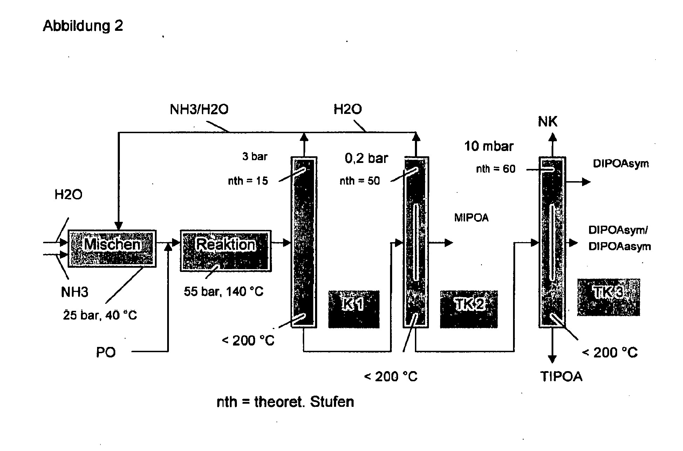

Demgemäß wurde ein Verfahren zur kontinuierlichen destillativen Auftrennung eines Gemisches enthaltend Monoisopropanolamin (MIPOA), Diisopropanolamin (DIPOA) und Triisopropanolamin (TIPOA) gefunden, welches dadurch gekennzeichnet ist, dass in einer konventionellen Destillationskolonne (K 1) Leichtersieder über Kopf und ein Gemisch enthaltend die Isopropanolamine über Sumpf abgetrennt werden, wobei Letzteres einer ersten Trennwandkolonne (TK 2) weiter aufgearbeitet wird, in der MIPOA als Seitenabzugsstrom aus dem längsunterteilten Bereich und ein Gemisch enthaltend DIPOA und TIPOA über Sumpf abgetrennt werden, wobei Letzteres in einer zweiten Trennwandkolonne (TK 3) weiter aufgearbeitet wird, in der DIPOA als Seitenabzugsstrom aus dem längsunterteilten Bereich und TIPOA über Sumpf gewonnen werden. Siehe Abbildung 2.

In alternativen Ausführungsformen kann anstelle einer Trennwandkolonne eine Zusammenschaltung von zwei (konventionellen) Destillationskolonnen in Form einer thermischen Kopplung verwendet werden.

Bei den ab- und aufzutrennenden Alkanolaminen handelt es sich um Monoisopropanolamin (MIPOA), Diisopropanolamin (DIPOA) und Triisopropanolamin (TIPOA).

Die Herstellung dieser Gemische kann nach verschiedenen in der Fachliteratur beschriebenen Verfahren erfolgen. Bei den Herstellverfahren wird Ammoniak z. B. im einbis zwanzigfachen molaren Überschuss bezogen auf Propylenoxid eingesetzt. Das Austragsgemisch dieser Umsetzungen, bestehend überwiegend aus ggf. unumgesetztem Ammoniak, ggf. Wasser, einem oder mehreren Alkanolaminen und Nebenprodukten, wird zunächst entspannt und ausgegast, anschließend werden Ammoniak und Wasser, jeweils teilweise oder vollständig, destillativ abgetrennt.

Es wurde gefunden, dass die weitere destillative Aufarbeitung und spezifikationsgerechte Reingewinnung des Alkanolamis oder der Alkanolamine besonders vorteilhaft hinsichtlich der Produktqualität gelingt, wenn die Aufarbeitung in Trennwandkolonnen oder thermisch gekoppelten Destillationskolonnen erfolgt und das/die Alkanolamin/e als Seitenfraktion/en entnommen werden. Diese Verfahrensgestaltung ermöglicht zudem niedrige Investitionskosten und einen geringen Energieverbrauch.

Eine typische, im erfindungsgemäßen Verfahren anzuwendende Trennwandkolonne (TK) (siehe Abbildung 1) weist jeweils eine Trennwand (T) in Kolonnenlängsrichtung unter Ausbildung eines oberen gemeinsamen Kolonnenbereichs (1), einen unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (5) und Abtriebsteil (3) auf, wobei die Zuführung des aufzutrennenden Gemischs (Feed) im mittleren Bereich des Zulaufteils (2, 4), die Abführung der Hochsiederfraktion über Sumpf (Sumpfabzug C), die Abführung der Leichtsiederfraktion über Kopf (Kopfabzug A) und die Abführung der Mittelsiederfraktion aus dem mittleren Bereich des Entnahmeteils (3, 5) (Seitenabzug B) erfolgt.

Die Trennwandkolonnen des erfindungsgemäßen Verfahrens weisen jeweils bevorzugt 30 bis 100, insbesondere 50 bis 90, theoretische Trennstufen auf.

Das Gemisch enthaltend Alkanolamine wird in Trennwandkolonnen aufgearbeitet, in denen das/die Alkanolamin/e als Seitenabzugsprodukte bevorzugt mit einer Reinheit > 98,0 Gew.-%, besonders ≥ 99,0 % Gew.-%, gewonnen werden.

Der Betriebsdruck der Kolonnen liegt bevorzugt im Bereich von 0,001 bis 5 bar, besonders bevorzugt 0,01 bis 2 bar, weiter besonders bevorzugt 0,1 bis 1,6 bar.

Unter Betriebsdruck ist in diesem Dokument der am Kopf der Kolonne gemessene absolute Druck zu verstehen.

Die Auftrennung wird in zwei hintereinandergeschalteten Trennwandkolonnen durchgeführt, wobei der Sumpfabzugstrom der ersten Trennwandkolonne den Zulaufstrom für die zweite Trennwandkolonne bildet.

Den Trennwandkolonnen (TK) ist eine konventionelle Destillationskolonne (K) vorgeschaltet, in der Leichtersieder über Kopf abgetrennt werden, wobei der Sumpfabzugstrom den Zulaufstrom für die erste Trennwandkolonne bildet.

Insbesondere weist im erfindungsgemäßen Verfahren der obere gemeinsame Kolonnenbereich (1) der Trennwandkolonnen (TK) 5 bis 50 %, bevorzugt 20 bis 35 %, der Verstärkungsteil (2) des Zulaufteils (2, 4) der Kolonne 5 bis 50 %, bevorzugt 10 bis 20 %, der Abtriebsteil (4) des Zulaufteils der Kolonne 5 bis 50 %, bevorzugt 20 bis 35 %, der Verstärkungsteil (3) des Entnahmeteils (3, 5) der Kolonne 5 bis 50 %, bevorzugt 7 bis 20 %, der Abtriebsteil (5) des Entnahmeteils der Kolonne 5 bis 50 %, bevorzugt 20 bis 35 %, und der gemeinsame untere Bereich (6) der Kolonne 5 bis 50 %, bevorzugt 20 bis 35 %, der Gesamtzahl der theoretischen Trennstufen (nth) der Kolonne auf.

Insbesondere beträgt in den Trennwandkolonnen (TK) jeweils die Summe der Zahl der theoretischen Trennstufen der Teilbereiche (2) und (4) im Zulaufteil 80 bis 110 %, bevorzugt 90 bis 100 %, der Summe der Zahl der Trennstufen der Teilbereiche (3) und (5) im Entnahmeteil.

Das erfindungsgemäße Verfahren ist bevorzugt dadurch gekennzeichnet, dass die Zulaufstelle und die Seitenabzugsstelle der Trennwandkolonnen zur Abtrennung von Alkanolamin/en hinsichtlich der Lage der theoretischen Trennstufen auf unterschiedlicher Höhe in der Kolonne angeordnet sind, indem sich die Zulaufstelle um 1 bis 20, insbesondere 5 bis 15, theoretische Trennstufen von der Seitenabzugsstelle unterscheidet.

Falls besonders hohe Anforderungen an die Reinheiten der Produkte gestellt werden, ist es günstig, die Trennwand mit einer thermischen Isolierung auszustatten. Eine Beschreibung der verschiedenen Möglichkeiten der thermischen Isolierung der Trennwand findet sich z. B. in EP 640 367 A. Eine doppelwandige Ausführung mit einem zwischenliegenden engen Gasraum ist besonders günstig.

Bevorzugt ist der durch die Trennwand (T) unterteilte Teilbereich der Trennwandkolonnen (TK) bestehend aus den Teilbereichen 2, 3, 4 und 5 oder Teilen davon mit geordneten Packungen oder Füllkörpern bestückt und die Trennwand in diesen Teilbereichen bevorzugt wärmeisolierend ausgeführt.

Im erfindungsgemäßen Verfahren wird/werden das Alkanolamin bzw. die Alkanolamine an der Seitenabzugsstelle in flüssiger Form oder gasförmig entnommen.

Bevorzugt wird der Brüdenstrom am unteren Ende der Trennwände (T) durch die Wahl und/oder Dimensionierung der Trenneinbauten und/oder den Einbau druckverlusterzeugender Vorrichtungen, beispielsweise von Blenden, so eingestellt, dass das Verhältnis des Brüdenstroms im Zulaufteil zu dem des Entnahmeteils 0,8 bis 1,2, insbesondere 0,9 bis 1,1, beträgt.

Die in diesem Dokument genannten Verhältnisse bezüglich bestimmter Ströme (z. B. Flüssigkeitsströme, Brüdenströme, Sumpfströme, Zulaufströme, Seitenabzugsströme) beziehen sich auf das Gewicht.

Bevorzugt wird die aus dem oberen gemeinsamen Bereich (1) der Trennwandkolonnen ablaufende Flüssigkeit in einem in der Kolonne oder außerhalb der Kolonne angeordneten Auffangraum gesammelt und gezielt durch eine Festeinstellung oder Regelung am oberen Ende der Trennwand (T) so aufgeteilt, dass das Verhältnis des Flüssigkeitsstroms zum Zulaufteil zu dem zum Entnahmeteil 0,1 bis 2,0, insbesondere 0,1 bis 1,0, z. B. 0,25 bis 0,8, beträgt.

Im erfindungsgemäßen Verfahren wird bevorzugt die Flüssigkeit auf den Zulaufteil 2 über eine Pumpe gefördert oder über eine statische Zulaufhöhe von mindestens 1 m mengengeregelt aufgegeben und die Regelung so eingestellt, dass die auf den Zulaufteil aufgegebene Flüssigkeitsmenge nicht unter 30 % des Normalwertes sinkt.

Im erfindungsgemäßen Verfahren wird bevorzugt die Aufteilung der aus dem Teilbereich 3 im Entnahmeteil der Trennwandkolonne ablaufenden Flüssigkeit auf den Seitenabzug und auf den Teilbereich 5 im Entnahmeteil der Kolonne durch eine Regelung, z. B. im Prozessleitsystem, so eingestellt, dass die auf den Teilbereich 5 aufgegebene Flüssigkeitsmenge nicht unter 30 % des Normalwertes sinkt.

Bevorzugt ist weiterhin, dass die Trennwandkolonnen (TK) am oberen und unteren Ende der Trennwand (T) Probenahmemöglichkeiten aufweisen und aus den Kolonnen kontinuierlich oder in zeitlichen Abständen flüssig oder gasförmig Proben entnommen und hinsichtlich ihrer Zusammensetzung untersucht werden.

Bei der Trennung von Mehrstoffgemischen in eine Leichtsieder-, Mittelsieder- und Hochsiederfraktion existieren üblicherweise Spezifikationen über den maximal zulässigen Anteil an Leichtsiedern und Hochsiedern in der Mittelsiederfraktion. Hierbei werden entweder einzelne für das Trennproblem kritische Komponenten, sogenannte Schlüsselkomponenten, oder die Summe von mehreren Schlüsselkomponenten spezifiziert.

Die Einhaltung der Spezifikation für die Hochsieder in der Mittelsiederfraktion wird bevorzugt über das Aufteilungsverhältnis der Flüssigkeit am oberen Ende der Trennwand geregelt. Dabei wird das Aufteilungsverhältnis der Flüssigkeit am oberen Ende der Trennwände (T) so eingestellt, dass die Konzentration der Schlüsselkomponenten für die Hochsiederfraktion in der Flüssigkeit am oberen Ende der Trennwand 5 bis 75 %, bevorzugt 10 bis 40 %, des Wertes ausmacht, der im Seitenabzugsprodukt erzielt werden soll, und die Flüssigkeitsaufteilung dahingehend eingestellt wird, dass bei höheren Gehalten an Schlüsselkomponenten der Hochsiederfraktion mehr und bei niedrigeren Gehalten an Schlüsselkomponenten der Hochsiederfraktion weniger Flüssigkeit auf den Zulaufteil geleitet wird.

Entsprechend wird die Spezifikation für die Leichtsieder in der Mittelsiederfraktion bevorzugt durch die Heizleistung geregelt. Hierbei wird die Heizleistung im Verdampfer der jeweiligen Trennwandkolonne so eingestellt, dass die Konzentration an Schlüsselkomponenten der Leichtsiederfraktion in der Flüssigkeit am unteren Ende der Trennwände (T) 10 bis 99 %, bevorzugt 25 bis 97,5 %, des Wertes ausmacht, der im Seitenabzugsprodukt erzielt werden soll, und die Heizleistung dahingehend eingestellt wird, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Zur Kompensation von Störungen der Zulaufmenge oder der Zulaufkonzentration erweist es sich zudem als vorteilhaft, durch entsprechende Regelvorschriften, z. B. im Prozessleitsystem, sicherzustellen, dass die Mengenströme der Flüssigkeiten, die auf die Kolonnenteile 2 und 5 (vgl. Abbildung 1) aufgegeben werden, nicht auf unter 30 % ihres Normalwertes sinken.

Zur Entnahme und Aufteilung der Flüssigkeiten am oberen Ende der Trennwand und an der Seitenentnahmestelle eignen sich sowohl innenliegende als auch außerhalb der Kolonne angeordnete Auffangräume für die Flüssigkeit, die die Funktion einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglichen. Bei der Verwendung von gepackten Kolonnen wird die Flüssigkeit zunächst in Sammlern gefasst und von dort aus in einen innenliegenden oder außenliegenden Auffangraum geleitet.

Das erfindungsgemäße Verfahren ist bevorzugt dadurch gekennzeichnet, dass die Destillatentnahme temperaturgeregelt erfolgt und als Regeltemperatur eine Messstelle im Teilbereich 1 der Trennwandkolonne verwendet wird, die um 2 bis 20, insbesondere 4 bis 15, theoretische Trennstufen unterhalb des oberen Endes der Kolonne angeordnet ist.

Das erfindungsgemäße Verfahren ist bevorzugt dadurch gekennzeichnet, dass die Entnahme des Sumpfprodukts temperaturgeregelt erfolgt und als Regeltemperatur eine Messstelle im Teilbereich 6 der Trennwandkolonne verwendet wird, die um 2 bis 20, insbesondere 4 bis 15, theoretische Trennstufen oberhalb des unteren Endes der Kolonne angeordnet ist.

In einer weiteren besonderen Ausgestaltung erfolgt die Entnahme des Seitenprodukts im Seitenabzug standgeregelt und als Regelgröße wird der Flüssigkeitsstand im Verdampfer verwendet.

Bevorzugt ist, dass die Trennwände nicht in die Kolonne eingeschweißt sind, sondern in Form von lose gesteckten und adäquat abgedichteten Teilsegmenten gestaltet sind.

Eine weitere erfindungsgemäße Variation des Verfahrens zur destillativen Aufarbeitung der Alkanolamine besteht darin, dass anstelle einer der genannten Trennwandkolonnen - die bei einem Neubau hinsichtlich der Investitionskosten zu bevorzugen sind - eine Zusammenschaltung von zwei (konventionellen) Destillationskolonnen in Form einer thermischen Kopplung verwendet wird (thermisch gekoppelte Kolonnen, die hinsichtlich des Energiebedarfs einer Trennwandkolonne entsprechen).
Dies ist vor allem dann günstig, wenn die Kolonnen schon vorhanden sind und/oder die Kolonnen bei verschiedenen Drücken betrieben werden sollen.

Je nach der Trennstufenzahl der vorhandenen Kolonnen können die geeignetsten Formen der Zusammenschaltung gewählt werden.
Bevorzugt sind beide thermisch gekoppelten Destillationskolonnen jeweils mit einem eigenen Verdampfer und Kondensator ausgestattet.

Weiterhin werden bevorzugt die beiden thermisch gekoppelten Kolonnen bei verschiedenen Drücken betrieben und in den Verbindungsströmen zwischen den beiden Kolonnen nur Flüssigkeiten gefördert.
Es ist also möglich, Schaltungsformen zu wählen, die es erlauben, dass nur flüssige Verbindungsströme zwischen den einzelnen Destillationskolonnen auftreten. Diese speziellen Verschaltungen bieten den Vorteil, dass die beiden Destillationskolonnen unter verschiedenen Drücken betrieben werden können mit dem Vorteil, dass sie sich besser an die Temperaturniveaus vorhandener Heiz- und Kühlenergien anpassen lassen.

Bevorzugt wird der Sumpfstrom der ersten Kolonne der beiden thermisch gekoppelten Kolonnen in einem zusätzlichen Verdampfer teilweise oder vollständig verdampft und anschließend der zweiten Kolonne zweiphasig oder in Form eines gasförmigen und eines flüssigen Stromes zugeführt.

Im erfindungsgemäßen Verfahren wird bevorzugt der Zulaufstrom (Feed) zur Kolonne/zu den Kolonnen teilweise oder vollständig vorverdampft und der/den Kolonne/n zweiphasig oder in Form eines gasförmigen und eines flüssigen Stromes zugeführt.

Trennwandkolonnen und thermisch gekoppelte Kolonnen können als Packungskolonnen mit Füllkörpern oder geordneten Packungen oder als Bodenkolonnen ausgeführt werden.

Bei der erfindungsgemäßen Reindestillation der Alkanolamine, die bevorzugt im Vakuum betrieben wird, empfiehlt es sich, Packungskolonnen einzusetzen. Dabei sind geordnete Blechpackungen mit einer spezifischen Oberfläche von 100 bis 500 m²/m³, bevorzugt etwa 250 bis 350 m²/m³, besonders geeignet.

Gegenstand der vorliegenden Erfindung ist auch eine Vorrichtung zur kontinuierlichen destillativen Auftrennung eines Gemisches enthaltend Monoisopropanolamin (MIPOA), Diisopropanolamin (DIPOA) und Triisopropanolamin (TIPOA), gekennzeichnet durch eine Ausgestaltung und Kolonnenverschaltung wie vorstehend und insbesondere in dem weiter unten aufgeführten Beispiel definiert und beschrieben.

In einer besonderen Ausgestaltung betrifft die Erfindung eine Vorrichtung, geeignet zur Auftrennung eines Gemisches enthaltend Monoisopropanolamin (MIPOA), Diisopropanolamin (DIPOA) und Triisopropanolamin (TIPOA), mit einer konventionellen Destillationskolonne (K 1), aufweisend einen Zulauf im mittleren Bereich, einen Kopfabzug, der bevorzugt zur Umsetzung von PO mit Ammoniak zurückführt, einen Sumpfabzug, einer Zuführung des Sumpfabzugs im längsunterteilten Bereich einer Trennwandkolonne (TK 2), aufweisend einen Seitenabzug für MIPOA im längsunterteilten Bereich, einen Kopfabzug, der bevorzugt zur Umsetzung von PO mit Ammoniak zurückführt, einen Sumpfabzug, einer Zuführung des Sumpfabzugs von TK 2 im längsunterteilten Bereich einer Trennwandkolonne (TK 3), aufweisend einen Seitenabzug für DIPOA-sym/asym-Gemisch im längsunterteilten Bereich, einen Seitenabzug für DIPOA-sym im oberen Kolonnenbereich (1), einen Kopfabzug und einen Sumpfabzug für TIPOA. Siehe Abbildung 2.

### Beispiel

Die APHA-Messungen erfolgten gemäß DIN ISO 6271.

Die Bestimmung von Wasser-Gehalten erfolgte standardgemäß durch Karl-Fischer-Titration.

Die Ermittlung der Reinheit von Alkanolaminen erfolgte nach vorheriger Derivatisierung mit Trifluoressigsäureanhydrid mittels Gaschromatographie wie folgt:
IPOA: Säule: CP SIL 8 CB - 25 m - 0,32 mm - 5 µm FD

Alle ppm-Angaben beziehen sich auf das Gewicht (Gew.-ppm).

Auftrennung eines Gemisches enthaltend die Isopropanolamine (IPOA) Monoisopropanolamin (MIPOA), Diisopropanolamine (DIPOAsym und DIPOAasym) und Triisopropanolamin (TIPOA)

### Siehe die Abbildung 2.

Im Mischkreis (25 bar, 40°C) werden NH₃ und Wasser gemischt und zusammen mit Propylenoxid (PO) dem Rohrreaktor (55 bar, 110-140 °C) zugeführt. Die wasserkatalysierte Synthese in der Flüssigphase von NH₃ mit PO zu IPOA läuft exotherm über eine Reihe von irreversiblen Folgereaktionen von NH₃ über MIPOA und DIPOA zu TIPOA. Der Rohrreaktor ist in mehrere Kühl- und Heizzonen unterteilt. Zur Abfuhr der Reaktionswärme wird der Reaktor im ersten Abschnitt mit Wasser gekühlt und zum vollständigen Reaktionsablauf (< 1 ppm PO) gegen Rohrende durch Beheizen auf Reaktionstemperatur gehalten. Die Reaktion wird üblicherweise mit einem molaren NH₃-Überschuss zwischen 3 und 8 betrieben, um gezielt das gewünschte Produktmix MIPOA/DIPOA/TIPOA zu erzielen. Im Mischkreis werden ca. 20 Gew.-% Wasser eingestellt. Aus dem Reaktionsgemisch werden in der Druckkolonne K 1 (15 theoretische Trennstufen) Wasser und überschüssiges NH₃ über Kopf bei einem Kolonnendruck von 3 bar abgetrennt und zum Mischbehälter zurückgeführt. Der Sumpf der K 1 bildet den Zulauf zur 1. Trennwandkolonne TK 2 (50 theoretische Trennstufen). In der TK 2 wird bei 200 mbar Kopfdruck reines MIPOA über einen flüssigen Seitenabzug jenseits der Trennwand gewonnen und restliches Wasser über Kopf recycelt. In der 2. Trennwandkolonne TK 3 (60 theoretische Trennstufen) werden simultan bei 10 mbar Kopfdruck reines DIPOAsym (sym = symmetrisch) über einen flüssigen Seitenabzug im trennwandfreien Verstärkungsteil reindestilliert, eine vorgegebene DI-POAasym/DIPOAsym-Mischung (asym = asymmetrisch) über einen flüssigen Seitenabzug jenseits der Trennwand gewonnen und reines TIPOA über Sumpf der TK 3 abgetrennt. Über Kopf der TK 3 werden Nebenkomponenten (NK) ausgeschleust. Um die spezifizierten Farbqualitäten der Isopropanolamine (IPOA) zu gewährleisten, sollen in den Kolonnen K 1, TK 2 und TK 3 Sumpftemperaturen von 200 °C nicht überschritten werden.

### Spezifikationen:

### MIPOA:

| | |
|---|---|
| Reinheit | > 99 Gew.-% |
| Diisopropanolamin: | < 0,1 Gew.-% |
| Wasser: | < 0,15 Gew.-% |
| Farbzahl: | < 20 APHA |

### DIPOAsym:

| | |
|---|---|
| Reinheit: | > 99 Gew.-% |
| Diisopropanolamin asym: | < 0,1 Gew.-% |
| Wasser: | < 0,1 Gew.-% |
| Farbzahl: | < 40 APHA |

### Mischung DIPOA sym/asym:

| | |
|---|---|
| Reinheit: | > 99 Gew.-% |
| Monoisopropanolamin: | < 0,9 Gew.-% |
| Wasser: | < 0,5 Gew.-% |
| Farbzahl: | < 40 APHA |

### TIPOA:

| | |
|---|---|
| Reinheit: | > 97 Gew.-% |
| Diisopropanolamin: | < 0,5 Gew.-% |
| Wasser: | < 0,5 Gew.-% |
| Farbzahl: | < 150 APHA |

Zusätzlich zur Variation des NH₃-Überschusses kann der Produktmix auch durch gezieltes Rückführen von DIPA in Richtung TIPA verschoben werden (TIPA-reiche Fahrweise).

## Patentansprüche

1. Verfahren zur kontinuierlichen destillativen Auftrennung eines Gemisches enthaltend Monoisopropanolamin (MIPOA), Diisopropanolamin (DIPOA) und Triisopropanolamin (TIPOA), **dadurch gekennzeichnet, dass** in einer konventionellen Destillationskolonne (K 1) Leichtersieder über Kopf und ein Gemisch enthaltend die Isopropanolamine über Sumpf abgetrennt werden, wobei Letzteres einer ersten Trennwandkolonne (TK 2) weiter aufgearbeitet wird, in der MIPOA als Seitenabzugsstrom aus dem längsunterteilten Bereich und ein Gemisch enthaltend DIPOA und TIPOA über Sumpf abgetrennt werden, wobei Letzteres in einer zweiten Trennwandkolonne (TK 3) weiter aufgearbeitet wird, in der DIPOA als Seitenabzugsstrom aus dem längsunterteilten Bereich und TIPOA über Sumpf gewonnen werden.

2. Verfahren nach Anspruch 1, **dadurch gekennzeichnet, dass** es sich bei dem Gemisch enthaltend die Alkanolamine um ein Produkt, erhalten durch Umsetzung von Propylenoxid (PO) mit Ammoniak und nachfolgender teilweiser oder vollständiger Abtrennung von unumgesetztem Ammoniak sowie gegebenenfalls Wasser, handelt.

3. Verfahren nach einem der vorhergehenden Ansprüche, **dadurch gekennzeichnet, dass** der Betriebsdruck der Kolonnen im Bereich von 0,001 bis 5 bar liegt.

4. Verfahren nach einem der vorhergehenden Ansprüche, **dadurch gekennzeichnet, dass** die Trennwandkolonne (TK) jeweils eine Trennwand (T) in Kolonnenlängsrichtung unter Ausbildung eines oberen gemeinsamen Kolonnenbereichs (1), einen unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (5) und Abtriebsteil (3) aufweist, wobei die Zuführung des aufzutrennenden Gemischs (Feed) im mittleren Bereich des Zulaufteils (2, 4), die Abführung der Hochsiederfraktion über Sumpf (Sumpfabzug C), die Abführung der Leichtsiederfraktion über Kopf (Kopfabzug A) und die Abführung der Mittelsiederfraktion aus dem mittleren Bereich des Entnahmeteils (3, 5) (Seitenabzug B) erfolgt.

5. Verfahren nach einem der vorhergehenden Ansprüche, **dadurch gekennzeichnet, dass** die Trennwandkolonnen jeweils 30 bis 100 theoretische Trennstufen aufweisen.

6. Verfahren nach einem der vorhergehenden Ansprüche 4 bis 5, **dadurch gekennzeichnet, dass** in den Trennwandkolonnen (TK) jeweils die Summe der Zahl der theoretischen Trennstufen der Teilbereiche (2) und (4) im Zulaufteil 80 bis 110 % der Summe der Zahl der Trennstufen der Teilbereiche (3) und (5) im Entnahmeteil beträgt.

7. Verfahren nach einem der Ansprüche 4 bis 6, **dadurch gekennzeichnet, dass** der obere gemeinsame Kolonnenbereich (1) der Trennwandkolonnen (TK) zur Abtrennung von Alkanolamin bzw. Alkanolaminen 5 bis 50 %, der Verstärkungsteil (2) des Zulaufteils (2, 4) der Trennwandkolonne 5 bis 50 %, der Abtriebsteil (4) des Zulaufteils der Trennwandkolonne 5 bis 50 %, der Verstärkungsteil (3) des Entnahmeteils (3, 5) der Trennwandkolonne 5 bis 50 %, der Abtriebsteil (5) des Entnahmeteils der Trennwandkolonne 5 bis 50 %, und der gemeinsame untere Bereich (6) der Kolonne 5 bis 50 % der Gesamtzahl der theoretischen Trennstufen der Trennwandkolonne aufweist.

8. Verfahren nach einem der Ansprüche 4 bis 7, **dadurch gekennzeichnet, dass** die Zulaufstelle und die Seitenabzugsstelle der Trennwandkolonnen zur Abtrennung von Alkanolamin/en hinsichtlich der Lage der theoretischen Trennstufen auf unterschiedlicher Höhe in der Kolonne angeordnet sind, indem sich die Zulaufstelle um 1 bis 20 theoretische Trennstufen von der Seitenabzugsstelle unterscheidet.

9. Verfahren nach einem der Ansprüche 4 bis 8, **dadurch gekennzeichnet, dass** der durch die Trennwand (T) unterteilte Teilbereich der Trennwandkolonnen (TK) bestehend aus den Teilbereichen 2, 3, 4 und 5 oder Teilen davon mit geordneten Packungen oder Füllkörpern bestückt ist und die Trennwand in diesen Teilbereichen wärmeisolierend ausgeführt ist.

10. Verfahren nach einem der vorhergehenden Ansprüche, **dadurch gekennzeichnet, dass** das Alkanolamin bzw. die Alkanolamine an der/den Seitenabzugsstelle/n in flüssiger Form entnommen wird/werden.

11. Verfahren nach einem der Ansprüche 1 bis 9, **dadurch gekennzeichnet, dass** das Alkanolamin bzw. die Alkanolamine an der/den Seitenabzugsstelle/n gasförmig entnommen wird/werden.

12. Verfahren nach einem der Ansprüche 4 bis 11, **dadurch gekennzeichnet, dass** der Brüdenstrom am unteren Ende der Trennwände (T) durch die Wahl und/oder Dimensionierung der Trenneinbauten und/oder den Einbau druckverlusterzeugender Vorrichtungen so eingestellt wird, dass das Verhältnis des Brüdenstroms im Zulaufteil zu dem des Entnahmeteils 0,8 bis 1,2 beträgt.

13. Verfahren nach einem der Ansprüche 4 bis 12, **dadurch gekennzeichnet, dass** die aus dem oberen gemeinsamen Bereich (1) der Trennwandkolonnen ablaufende Flüssigkeit in einem in der Kolonne oder außerhalb der Kolonne angeordneten Auffangraum gesammelt und gezielt durch eine Festeinstellung oder Regelung am oberen Ende der Trennwand (T) so aufgeteilt wird, dass das Verhältnis des Flüssigkeitsstroms zum Zulaufteil zu dem zum Entnahmeteil 0,1 bis 2,0 beträgt.

14. Verfahren nach einem der Ansprüche 4 bis 13, **dadurch gekennzeichnet, dass** die Flüssigkeit auf den Zulaufteil 2 über eine Pumpe gefördert oder über eine statische Zulaufhöhe von mindestens 1 m mengengeregelt aufgegeben wird und die Regelung so eingestellt wird, dass die auf den Zulaufteil aufgegebene Flüssigkeitsmenge nicht unter 30 % des Normalwertes sinkt.

15. Verfahren nach einem der Ansprüche 4 bis 14, **dadurch gekennzeichnet, dass** die Aufteilung der aus dem Teilbereich 3 im Entnahmeteil der Trennwandkolonne ablaufenden Flüssigkeit auf den Seitenabzug und auf den Teilbereich 5 im Entnahmeteil der Kolonne durch eine Regelung so eingestellt wird, dass die auf den Teilbereich 5 aufgegebene Flüssigkeitsmenge nicht unter 30 % des Normalwertes sinkt.

16. Verfahren nach einem der vorhergehenden Ansprüche, **dadurch gekennzeichnet, dass** das Aufteilungsverhältnis der Flüssigkeit am oberen Ende der Trennwände (T) so eingestellt wird, dass die Konzentration an denjenigen Komponenten der Hochsiederfraktion, für die im Seitenabzug ein bestimmter Grenzwert für die Konzentration erzielt werden soll, in der Flüssigkeit am oberen Ende der Trennwand 5 bis 75 % des Wertes ausmacht, der im Seitenabzugsprodukt erzielt werden soll, und die Flüssigkeitsaufteilung dahingehend eingestellt wird, dass bei höheren Gehalten an Komponenten der Hochsiederfraktion mehr und bei niedrigeren Gehalten an Komponenten der Hochsiederfraktion weniger Flüssigkeit auf den Zulaufteil geleitet wird.

17. Verfahren nach einem der vorhergehenden Ansprüche, **dadurch gekennzeichnet, dass** die Heizleistung im Verdampfer so eingestellt wird, dass die Konzentration an denjenigen Komponenten der Leichtsiederfraktion, für die im Seitenabzug ein bestimmter Grenzwert für die Konzentration erzielt werden soll, am unteren Ende der Trennwände (T) so eingestellt wird, dass die Konzentration an Komponenten der Leichtsiederfraktion in der Flüssigkeit am unteren Ende der Trennwand 10 bis 99 % des Wertes ausmacht, der im Seitenabzugsprodukt erzielt werden soll, und die Heizleistung dahingehend eingestellt wird, dass bei höherem Gehalt an Komponenten der Leichtsiederfraktion die Heizleistung erhöht und bei niedrigerem Gehalt an Komponenten der Leichtsiederfraktion die Heizleistung verringert wird.

18. Verfahren nach einem der Ansprüche 4 bis 17, **dadurch gekennzeichnet, dass** die Destillatentnahme temperaturgeregelt erfolgt und als Regeltemperatur eine Messstelle im Teilbereich 1 der Kolonne verwendet wird, die um 2 bis 20 theoretische Trennstufen unterhalb des oberen Endes der Trennwandkolonne angeordnet ist.

19. Verfahren nach einem der Ansprüche 4 bis 18, **dadurch gekennzeichnet, dass** die Entnahme des Sumpfprodukts temperaturgeregelt erfolgt und als Regeltemperatur eine Messstelle im Teilbereich 6 der Trennwandkolonne verwendet wird, die um 2 bis 20, theoretische Trennstufen oberhalb des unteren Endes der Trennwandkolonne angeordnet ist.

20. Verfahren nach einem der vorhergehenden Ansprüche, **dadurch gekennzeichnet, dass** die Entnahme des Alkanolamins im Seitenabzug standgeregelt erfolgt und als Regelgröße der Flüssigkeitsstand im Verdampfer verwendet wird.

21. Verfahren nach einem der vorhergehenden Ansprüche, **dadurch gekennzeichnet, dass** die Trennwände nicht in die Kolonnen eingeschweißt sind, sondern in Form von lose gesteckten und adäquat abgedichteten Teilsegmenten gestaltet sind.

22. Verfahren nach einem der vorhergehenden Ansprüche, **dadurch gekennzeichnet, dass** anstelle einer Trennwandkolonne eine Zusammenschaltung von zwei Destillationskolonnen in Form einer thermischen Kopplung verwendet wird.

23. Verfahren nach dem vorhergehenden Anspruch, **dadurch gekennzeichnet, dass** beide thermisch gekoppelten Destillationskolonnen jeweils mit einem eigenen Verdampfer und Kondensator ausgestattet sind.

24. Verfahren nach einem der beiden vorhergehenden Ansprüche, **dadurch gekennzeichnet, dass** die beiden thermisch gekoppelten Kolonnen bei verschiedenen Drücken betrieben werden und in den Verbindungsströmen zwischen den beiden Kolonnen nur Flüssigkeiten gefördert werden.

25. Verfahren nach einem der drei vorhergehenden Ansprüche, **dadurch gekennzeichnet, dass** der Sumpfstrom der ersten Kolonne in einem zusätzlichen Verdampfer teilweise oder vollständig verdampft wird und anschließend der zweiten Kolonne zweiphasig oder in Form eines gasförmigen und eines flüssigen Stromes zugeführt wird.

26. Verfahren nach einem der vorhergehenden Ansprüche, **dadurch gekennzeichnet, dass** der Zulaufstrom (Feed) zur Kolonne/zu den Kolonnen teilweise oder vollständig vorverdampft wird und der/den Kolonne/n zweiphasig oder in Form eines gasförmigen und eines flüssigen Stromes zugeführt wird.

27. Vorrichtung, geeignet zur kontinuierlichen destillativen Auftrennung eines Gemisches enthaltend Monoisopropanolamin (MIPOA), Diisopropanolamin (DIPOA) und Triisopropanolamin (TIPOA), umfassend eine konventionelle Destillationskolonne (K 1), aufweisend einen Zulauf im mittleren Bereich, einen Kopfabzug, einen Sumpfabzug, eine Zuführung des Sumpfabzugs im längsunterteilten Bereich einer Trennwandkolonne (TK 2), aufweisend einen Seitenabzug für MIPOA im längsunterteilten Bereich, einen Kopfabzug, einen Sumpfabzug, eine Zuführung des Sumpfabzugs von TK 2 im längsunterteilten Bereich einer Trennwandkolonne (TK 3), aufweisend einen Seitenabzug für DIPOA-sym/asym-Gemisch im längsunterteilten Bereich, einen Seitenabzug für DIPOA-sym im oberen Kolonnenbereich (1), einen Kopfabzug und einen Sumpfabzug für TIPOA.

## Claims

1. A process for continuous distillative separation of a mixture comprising monoisopropanolamine (MIPOA), diisopropanolamine (DIPOA) and triisopropanolamine (TIPOA), wherein, in a conventional distillation column (C 1), relatively low boilers are removed via the top and a mixture comprising the isopropanolamines is removed via the bottom, the latter being worked up further in a first dividing wall column (DWC 2) in which MIPOA is removed as a side draw stream from the longitudinally divided region and a mixture comprising DIPOA and TIPOA via the bottom, the latter being worked up further in a second dividing wall column (DWC 3) in which DIPOA is obtained as a side draw stream from the longitudinally divided region and TIPOA via the bottom.

2. The process according to claim 1, wherein the mixture comprising the alkanolamines is a product obtained by reacting propylene oxide (PO) with ammonia and subsequent partial or complete removal of unconverted ammonia, with or without water.

3. The process according to either of the preceding claims, wherein the operating pressure of the columns is in the range from 0.001 to 5 bar.

4. The process according to any one of the preceding claims, wherein the dividing wall column (DWC) in each case has a dividing wall (DW) in the longitudinal direction of the column to form an upper common column region (1), a lower common column region (6), a feed section (2, 4) comprising rectifying section (2) and stripping section (4), and a withdrawal section (3, 5) comprising rectifying section (5) and stripping section (3), the mixture to be separated (feed) being fed in in the middle region of the feed section (2, 4), the high boiler fraction being removed via the bottom (bottom draw C), the low boiler fraction being removed via the top (top draw A) and the medium boiler fraction being removed from the middle region of the withdrawal section (3, 5) (side draw B).

5. The process according to any one of the preceding claims, wherein the dividing wall columns each have 30 to 100 theoretical plates.

6. The process according to either of the preceding claims 4 and 5, wherein, in the dividing wall columns (DWC), the sum of the number of theoretical plates of subregions (2) and (4) in the feed section is in each case 80 to 110% of the sum of the number of plates of subregions (3) and (5) in the withdrawal section.

7. The process according to any one of claims 4 to 6, wherein the upper common column region (1) of the dividing wall columns (DWC) for removal of alkanolamine or alkanolamines has 5 to 50%, the rectifying section (2) of the feed section (2, 4) of the dividing wall column 5 to 50%, the stripping section (4) of the feed section of the dividing wall column 5 to 50%, the rectifying section (3) of the withdrawal section (3, 5) of the dividing wall column 5 to 50%, the stripping section (5) of the withdrawal section of the dividing wall column 5 to 50%, and the common lower region (6) of the column 5 to 50%, of the total number of theoretical plates of the dividing wall column.

8. The process according to any one of claims 4 to 7, wherein the feed point and the side draw point of the dividing wall columns for removal of alkanolamine(s), with regard to the position of the theoretical plates, are arranged at different heights in the column, by virtue of the feed point differing by 1 to 20 theoretical plates from the side draw point.

9. The process according to any one of claims 4 to 8, wherein the subregion of the dividing wall columns (DWC) which is divided by the dividing wall (DW) and consists of subregions 2, 3, 4 and 5 or parts thereof is equipped with structured packings or random packings, and the dividing wall is configured with thermal insulation in these subregions.

10. The process according to any one of the preceding claims, wherein the alkanolamine or the alkanolamines is/are withdrawn in liquid form at the side draw point(s).

11. The process according to any one of claims 1 to 9, wherein the alkanolamine or the alkanolamines is/are withdrawn in gaseous form at the side draw point(s).

12. The process according to any one of claims 4 to 11, wherein the vapor stream at the lower end of the dividing walls (DW) is adjusted through the selection and/or dimensions of the separating internals and/or the installation of pressure dropgenerating devices such that the ratio of the vapor stream in the feed section relative to that of the withdrawal section is 0.8 to 1.2.

13. The process according to any one of claims 4 to 12, wherein the liquid effluxing from the upper common region (1) of the dividing wall columns is collected in a collecting space arranged within the column or outside the column and is divided in a controlled manner by a fixed setting or regulator at the upper end of the dividing wall (DW) such that the ratio of the liquid stream to the feed section to that to the withdrawal section is 0.1 to 2.0.

14. The process according to any one of claims 4 to 13, wherein the liquid is conveyed to the feed section 2 by means of a pump or introduced under quantitative control by means of a static feed head of at least 1 m, and the regulator is adjusted such that the amount of liquid introduced to the feed section does not fall below 30% of the normal value.

15. The process according to any one of claims 4 to 14, wherein the division of the liquid effluxing from the subregion 3 in the withdrawal section of the dividing wall column between the side draw and the subregion 5 in the withdrawal section of the column is adjusted by a regulator such that the amount of liquid introduced to the subregion 5 does not fall below 30% of the normal value.

16. The process according to any one of the preceding claims, wherein the division ratio of the liquid at the upper end of the dividing walls (DW) is adjusted such that the concentration of those components of the high boiler fraction for which a particular concentration limit is to be achieved in the side draw, in the liquid at the upper end of the dividing wall, amounts to 5 to 75% of the value which is to be achieved in the side draw product, and the liquid division is adjusted to the effect that more liquid is passed to the feed section in the event of higher contents of components of the high boiler fraction, and less in the event of lower contents of components of the high boiler fraction.

17. The process according to any one of the preceding claims, wherein the heating output in the evaporator is adjusted such that the concentration of those components of the low boiler fraction for which a particular concentration limit is to be achieved in the side draw, at the lower end of the dividing walls (DW), is adjusted such that the concentration of components of the low boiler fraction in the liquid at the lower end of the dividing wall amounts to 10 to 99% of the value which is to be achieved in the side draw product, and the heating output is adjusted to the effect that the heating output is increased in the event of a higher content of components of the low boiler fraction and the heating output is reduced in the event of a lower content of components of the low boiler fraction.

18. The process according to any one of claims 4 to 17, wherein the distillate is withdrawn under thermal control and the control temperature used is a measurement point in the subregion 1 of the column, which is arranged 2 to 20 theoretical plates below the upper end of the dividing wall column.

19. The process according to any one of claims 4 to 18, wherein the bottom product is withdrawn under thermal control and the control temperature used is a measurement point in the subregion 6 of the dividing wall column, which is arranged 2 to 20 theoretical plates above the lower end of the dividing wall column.

20. The process according to any one of the preceding claims, wherein the alkanolamine is withdrawn in the side draw under level control and the control parameter used is the liquid level in the evaporator.

21. The process according to any one of the preceding claims, wherein the dividing walls are not welded into the columns but are instead configured in the form of loosely inserted and adequately sealed subsegments.

22. The process according to any one of the preceding claims, wherein a connection of two distillation columns in the form of a thermal coupling is used instead of a dividing wall column.

23. The process according to the preceding claim, wherein each thermally coupled distillation column is equipped with a dedicated evaporator and condenser.

24. The process according to either of the two preceding claims, wherein the two thermally coupled columns are operated at different pressures and only liquids are conveyed in the connecting streams between the two columns.

25. The process according to any one of the three preceding claims, wherein the bottom stream of the first column is evaporated partially or completely in an additional evaporator and then fed to the second column in biphasic form or in the form of a gaseous stream and of a liquid stream.

26. The process according to any one of the preceding claims, wherein the feed stream (feed) to the column/to the columns is partially or completely preevaporated and is fed to the column(s) in biphasic form or in the form of a gaseous stream and of a liquid stream.

27. An apparatus suitable for continuous distillative separation of a mixture comprising monoisopropanolamine (MIPOA), diisopropanolamine (DIPOA) and triisopropanolamine (TIPOA), comprising a conventional distillation column (K 1) having a feed in the middle region, a top draw, a bottom draw, a feed for the bottom draw in the longitudinally divided region of a dividing wall column (DWC 2) having a side draw for MIPOA in the longitudinally divided region, a top draw, a bottom draw, a feed for the bottom draw from DWC 2 in the longitudinally divided region of a dividing wall column (DWC 3) having a side draw for DIPOA-sym/asym mixture in the longitudinally divided region, a side draw for DIPOA-sym in the upper column region (1), a top draw and a bottom draw for TIPOA.

## Revendications

1. Procédé de séparation par distillation en continu d'un mélange contenant de la monoisopropanolamine (MIPOA), de la diisopropanolamine (DIPOA) et de la triisopropanolamine (TIPOA), **caractérisé en ce que**, dans une colonne de distillation classique (K 1), les composants de point d'ébullition faible sont séparés par la tête et un mélange contenant les isopropanolamines par le fond, ce dernier étant ensuite traité dans une première colonne à paroi de séparation (TK 2), dans laquelle le MIPOA est séparé en tant que courant de soutirage latéral à partir de la zone divisée longitudinalement et un mélange contenant le DIPOA et le TIPOA par le fond, ce dernier étant ensuite traité dans une seconde colonne à paroi de séparation (TK 3), dans laquelle le DIPOA est obtenu en tant que courant de soutirage latéral à partir de la zone divisée longitudinalement et le TIPOA par le fond.

2. Procédé selon la revendication 1, **caractérisé en ce que** le mélange contenant les alcanolamines est un produit obtenu par mise en réaction d'oxyde de propylène (PO) avec de l'ammoniac, puis séparation partielle ou complète de l'ammoniac non réagi et éventuellement de l'eau.

3. Procédé selon l'une quelconque des revendications précédentes, **caractérisé en ce que** la pression d'exploitation des colonnes se situe dans la plage allant de 0,001 à 5 bar.

4. Procédé selon l'une quelconque des revendications précédentes, **caractérisé en ce que** la colonne à paroi de séparation (TK) comprend à chaque fois une paroi de séparation (T) dans la direction longitudinale de la colonne, formant une zone de colonne commune supérieure (1), une zone de colonne commune inférieure (6), une zone d'alimentation (2, 4) comprenant une zone d'enrichissement (2) et une zone de rectification (4), ainsi qu'une zone de soutirage (3, 5) comprenant une zone d'enrichissement (5) et une zone de rectification (3), l'introduction du mélange à séparer (alimentation) ayant lieu dans la partie centrale de la zone d'alimentation (2, 4), le déchargement de la fraction de composants de point d'ébullition élevé ayant lieu par le fond (soutirage de fond C), le déchargement de la fraction de composants de point d'ébullition faible ayant lieu par la tête (sortie de tête A) et le déchargement de la fraction de composants de point d'ébullition intermédiaire ayant lieu par la partie centrale de la zone de soutirage (3, 5) (soutirage latéral B).

5. Procédé selon l'une quelconque des revendications précédentes, **caractérisé en ce que** les colonnes à paroi de séparation comprennent à chaque fois 30 à 100 étapes de séparation théoriques.

6. Procédé selon l'une quelconque des revendications 4 à 5 précédentes, **caractérisé en ce que**, dans les colonnes à paroi de séparation (TK), la somme du nombre d'étapes de séparation théoriques des zones (2) et (4) de la zone d'alimentation est à chaque fois de 80 à 110 % de la somme du nombre d'étapes de séparation des zones (3) et (5) de la zone de soutirage.

7. Procédé selon l'une quelconque des revendications 4 à 6, **caractérisé en ce que** la zone de colonne commune supérieure (1) des colonnes à paroi de séparation (TK) pour la séparation de l'alcanolamine ou des alcanolamines comprend 5 à 50 %, la zone d'enrichissement (2) de la zone d'alimentation (2, 4) de la colonne à paroi de séparation 5 à 50 %, la zone de rectification (4) de la zone d'alimentation de la colonne à paroi de séparation 5 à 50 %, la zone d'enrichissement (3) de la zone de soutirage (3, 5) de la colonne à paroi de séparation 5 à 50 %, la zone de rectification (5) de la zone de soutirage de la colonne à paroi de séparation 5 à 50 %, et la zone inférieure commune (6) de la colonne 5 à 50 % du nombre total d'étapes de séparation théoriques de la colonne à paroi de séparation.

8. Procédé selon l'une quelconque des revendications 4 à 7, **caractérisé en ce que** l'emplacement d'alimentation et l'emplacement de soutirage latéral des colonnes à paroi de séparation pour la séparation de la ou des alcanolamines sont agencés à une hauteur différente dans la colonne par rapport à la position des étapes de séparation théoriques, l'emplacement d'alimentation différant d'au moins 1 à 20 étapes de séparation théoriques de l'emplacement de soutirage latéral.

9. Procédé selon l'une quelconque des revendications 4 à 8, **caractérisé en ce que** la zone de la colonne à paroi de séparation (TK) divisée par la paroi de séparation (T) constituée des zones 2, 3, 4 et 5 ou de parties de celles-ci est équipée de garnissages structurés ou de corps de remplissage, et la paroi de séparation est configurée sous une forme isolée thermiquement dans ces zones.

10. Procédé selon l'une quelconque des revendications précédentes, **caractérisé en ce que** l'alcanolamine ou les alcanolamines sont soutirées sous forme liquide au niveau du ou des emplacements de soutirage latéraux.

11. Procédé selon l'une quelconque des revendications 1 à 9, **caractérisé en ce que** l'alcanolamine ou les alcanolamines sont soutirées sont forme gazeuse au niveau du ou des emplacements de soutirage latéraux.

12. Procédé selon l'une quelconque des revendications 4 à 11, **caractérisé en ce que** le courant de vapeur à l'extrémité inférieure des parois de séparation (T) est ajusté par le choix et/ou le dimensionnement des composants de séparation et/ou l'incorporation de dispositifs générant une perte de charge, de manière à ce que le rapport entre le courant de vapeur dans la zone d'alimentation et celui de la zone de soutirage soit de 0,8 à 1,2.

13. Procédé selon l'une quelconque des revendications 4 à 12, **caractérisé en ce que** le liquide s'écoulant de la zone commune supérieure (1) des colonnes à paroi de séparation est recueilli dans une chambre de recueillement agencée dans la colonne ou à l'extérieur de la colonne et divisé de manière ciblée par un ajustage fixe ou un réglage à l'extrémité supérieure de la paroi de séparation (T), de manière à ce que le rapport entre le courant de liquide dans la zone d'alimentation et celui dans la zone de soutirage soit de 0,1 à 2,0.

14. Procédé selon l'une quelconque des revendications 4 à 13, **caractérisé en ce que** le liquide est transporté dans la zone d'alimentation 2 par une pompe ou introduit avec régulation de la quantité par une hauteur d'alimentation statique d'au moins 1m, et le réglage est ajusté de manière à ce que la quantité de liquide introduite dans la zone d'alimentation ne chute pas en dessous de 30 % de la valeur normale.

15. Procédé selon l'une quelconque des revendications 4 à 14, **caractérisé en ce que** la division du liquide s'écoulant de la zone 3 dans la zone de soutirage de la colonne à paroi de séparation est ajustée dans la sortie latérale et dans la zone 5 de la zone de soutirage de la colonne par un réglage, de manière à ce que la quantité de liquide introduite dans la zone 5 ne chute pas en dessous de 30 % de la valeur normale.

16. Procédé selon l'une quelconque des revendications précédentes, **caractérisé en ce que** le rapport de partage du liquide à l'extrémité supérieure des parois de séparation (T) est ajusté de manière à ce que la concentration des composants de la fraction de composants de point d'ébullition élevé, pour lesquels une valeur limite déterminée pour la concentration doit être atteinte dans la sortie latérale, représente dans le liquide à l'extrémité supérieure de la paroi de séparation 5 à 75 % de la valeur qui doit être atteinte dans le produit de soutirage latéral, et le partage du liquide est pour cela ajusté en introduisant davantage de liquide dans la zone d'alimentation lorsque les teneurs en composants de la fraction de composants de point d'ébullition élevé sont plus élevées et moins lorsque les teneurs en composants de la fraction de composants de point d'ébullition élevé sont plus faibles.

17. Procédé selon l'une quelconque des revendications précédentes, **caractérisé en ce que** la puissance de chauffage dans l'évaporateur est ajustée de manière à ce que la concentration des composants de la fraction de composants de point d'ébullition faible, pour lesquels une valeur limite déterminée pour la concentration doit être atteinte dans la sortie latérale, soit ajustée à l'extrémité inférieure des parois de séparation (T) de manière à ce que la concentration des composants de la fraction de composants de point d'ébullition faible dans le liquide à l'extrémité inférieure de la paroi de séparation représente 10 à 99 % de la valeur qui doit être atteinte dans le produit de soutirage latéral, et la puissance de chauffage est pour cela ajustée en augmentant la puissance de chauffage lorsque la teneur en composants de la fraction de composants de point d'ébullition faible est plus élevée et en réduisant la puissance de chauffage lorsque la teneur en composants de la fraction de composants de point d'ébullition faible est plus faible.

18. Procédé selon l'une quelconque des revendications 4 à 17, **caractérisé en ce que** le soutirage de distillat a lieu avec régulation de la température, et un emplacement de mesure dans la zone 1 de la colonne est utilisé en tant que température de régulation, qui est agencé 2 à 20 étapes de séparation théoriques en-dessous de l'extrémité supérieure de la colonne à paroi de séparation.

19. Procédé selon l'une quelconque des revendications 4 à 18, **caractérisé en ce que** le soutirage du produit de fond a lieu avec régulation de la température, et un emplacement de mesure dans la zone 6 de la colonne à paroi de séparation est utilisé en tant que température de régulation, qui est agencé 2 à 20 étapes de séparation théoriques au dessus de l'extrémité inférieure de la colonne à paroi de séparation.

20. Procédé selon l'une quelconque des revendications précédentes, **caractérisé en ce que** le soutirage de l'alcanolamine dans la sortie latérale a lieu avec régulation du niveau, et le niveau de liquide dans l'évaporateur est utilisé en tant que grandeur de régulation.

21. Procédé selon l'une quelconque des revendications précédentes, **caractérisé en ce que** les parois de séparation ne sont pas soudées dans les colonnes, mais plutôt configurées sous la forme de segments partiels connectés de manière lâche et étanchéifiés de manière adéquate.

22. Procédé selon l'une quelconque des revendications précédentes, **caractérisé en ce qu'**un raccordement de deux colonnes de distillation sous la forme d'un couplage thermique est utilisé à la place d'une colonne à paroi de séparation.

23. Procédé selon la revendication précédente, **caractérisé en ce que** les deux colonnes de distillation couplées thermiquement sont chacune équipées d'un évaporateur et d'un condensateur propres.

24. Procédé selon l'une quelconque des deux revendications précédentes, **caractérisé en ce que** les deux colonnes couplées thermiquement sont exploitées à des pressions différentes et seuls des liquides sont transportés dans les courants de connexion entre les deux colonnes.

25. Procédé selon l'une quelconque des trois revendications précédentes, **caractérisé en ce que** le courant de fond de la première colonne est évaporé en partie ou en totalité dans un évaporateur supplémentaire, puis introduit dans la seconde colonne sous forme biphasée ou sous la forme d'un courant gazeux et d'un courant liquide.

26. Procédé selon l'une quelconque des revendications précédentes, **caractérisé en ce que** le courant d'alimentation (alimentation) de la colonne ou des colonnes est pré-évaporé en partie ou en totalité et introduit dans la ou les colonnes sous forme biphasée ou sous la forme d'un courant gazeux et d'un courant liquide.

27. Dispositif, approprié pour la séparation par distillation en continu d'un mélange contenant de la monoisopropanolamine (MIPOA), de la diisopropanolamine (DIPOA) et de la triisopropanolamine (TIPOA), comprenant une colonne de distillation classique (K 1), comprenant une alimentation dans la partie centrale, une sortie de tête, une sortie de fond, une alimentation de la sortie de fond dans la zone divisée longitudinalement d'une colonne à paroi de séparation (TK 2), comprenant une sortie latérale pour le MIPOA dans la zone divisée longitudinalement, une sortie de tête, une sortie de fond, une alimentation de la sortie de fond de TK 2 dans la zone divisée longitudinalement d'une colonne à paroi de séparation (TK 3), comprenant une sortie latérale pour un mélange DIPOA-sym/asym dans la zone divisée longitudinalement, une sortie latérale pour le DIPOA-sym dans la zone de colonne supérieure (1), une sortie de tête et une sortie de fond pour le TIPOA.
